**Europäisches Patentamt**

**European Patent Office**

**Office européen des brevets**

(19)

(11) Veröffentlichungsnummer: **0 011 866**

**A1**

# EUROPÄISCHE PATENTANMELDUNG

(12)

(21) Anmeldenummer: **79104777.2**

(22) Anmeldetag: **29.11.79**

(51) Int. Cl.³: **A 61 B 5/10**
**A 61 B 5/00**

(30) Priorität: **04.12.78 DE 2852351**

(43) Veröffentlichungstag der Anmeldung:
**11.06.80 Patentblatt 80/12**

(84) Benannte Vertragsstaaten:
**AT BE CH FR GB IT LU NL SE**

(71) Anmelder: **SIEMENS AKTIENGESELLSCHAFT Berlin
und München
Postfach 22 02 61
D-8000 München 22(DE)**

(72) Erfinder: **Reichenberger, Helmut, Dr. Dipl.-Phys.
Irisstrasse 8
D-8501 Brand/Eckental(DE)**

(72) Erfinder: **Walz, Volker, Dipl.-Ing.
Buckhower Damm 113
D-1000 Berlin 47(DE)**

(72) Erfinder: **Zachmann, Wilfried
Nöttinger Strasse 41
D-7537 Remchingen(DE)**

(54) **Vorrichtung zur Erfassung von Körperfunktionen von Patienten, insbesondere von Säuglingen.**

(57) Die Erfindung bezieht sich auf eine Vorrichtung zur Erfassung von Körperfunktionen von Patienten, insbesondere von Säuglingen, in Verbindung mit mechanischen Einwirkungen auf eine Lagerstaff für den Patienten, die mittels Kraftsignalgeber und zugeordneten Verarbeitungsgliedern erfaßbar sind. Es sind Überwachungsvorrichtungen bekannt, bei denen ein Kraftsignalgeber in ein Bein eines üblichen Klinikbettes eingebaut ist. Derartige Überwachungsvorrichtungen sind nur begrenzt einsetzbar. Gemäß der Erfindung ist die Lagerstatt für den Patienten aus einem Unterteil (1) und einem darauf gelagerten Oberteil (7) mit Liegefläche (8) für den Patienten gebildet, wobei die Lagerelemente (4 bis 6) zwischen Unterteil (1) und Oberteil (7) in wenigstens einem Einzelelement als Kraftsignalgeber (14,15) ausgebildet sind, und beinhaltet die Lagerstatt Mittel zur Unterdrückung von außen einwirkender, nicht vom Patienten verursachter Kräfte.

FIG 1

Croydon Printing Company Ltd.

SIEMENS AKTIENGESELLSCHAFT      Unser Zeichen

Berlin und München              VPA 78 P 5136 **EUR**

Vorrichtung zur Erfassung von Körperfunktionen von Patienten, insbesondere von Säuglingen

Die Erfindung bezieht sich auf eine Vorrichtung zur Erfassung von Körperfunktionen von Patienten, insbesondere von Säuglingen, in Verbindung mit mechanischen Einwirkungen auf eine Lagerstatt für den Patienten, die mittels Kraftsignalgeber und zugeordneten Verarbeitungsgliedern erfaßbar sind.

Die Überwachung von Patienten auf physiologische Körperfunktionen erfolgt in der Klinik meist mittels am Körper des Patienten angebrachter Meßwertabnehmer. Mit solchen Abnehmern, insbesondere Elektroden, können beispielsweise Herz- und Atmungsaktionen als elektrische Signale erfaßt werden, in einem Betriebsgerät nachverarbeitet und so bezüglich signifikanter Veränderungen der Körperfunktionen ausgewertet werden. Speziell nun die Säuglingsüberwachung mittels solcher Abnehmer ist

Wht 5 Kli / 29.11.1978

relativ aufwendig und daher nur im Klinikbereich, insbesondere auf der Frühgeborenen- bzw. Intensivstation, durchführbar. Es hat sich aber gezeigt, daß gerade die Überwachung von Säuglingen in viel größerem Maße als bisher wünschenswert wäre. Dies gilt insbesondere zur Bekämpfung des noch immer ungeklärten sogenannten "Krippentodes" (SID-Syndrom).

Es besteht also die Notwendigkeit, weitere einfach aufgebaute, universell verwendbare, aber trotzdem leistungsfähige Überwachungsvorrichtungen für Patienten zu schaffen, die speziell für Säuglinge verwendbar sind.

Zur Überwachung von Säuglingen ist bereits eine sogenannte "Apnoe-Matratze" bekannt (Med. & Biol.Eng. (1976), Seiten 306 bis 312). In eine solche luftgefüllte Matratze mit separaten Luftkammern ist ein Strömungsmeßfühler eingebaut, mit dem Lageveränderungen und auch insbesondere Atmungsaktivitäten erfaßt werden können. An gleicher Stelle sind weitere Anordnungen beschrieben, bei denen ein Kraftaufnehmer unmittelbar unter den Kopf oder Körper des Säuglings angeordnet ist. Nachteilig ist bei diesen Anordnungen, daß die Signale in starkem Maße abhängig von der relativen Lage von Meßwertaufnehmer und Säugling sind. Entsprechende Anordnungen zur berührungslosen Erfassung der Herzaktion von Säuglingen sind nicht bekannt.

Es wurde daneben auch schon versucht, durch Einbau von Kraftsignalgebern in die Beine gewöhnlicher Klinikbetten Körperfunktionssignale von Patienten abzuleiten (Biomed. Technik 21 (1976), Seiten 143 bis 147). Die Eigenart einer solchen Vorrichtung ist es aber, daß sie bevorzugt zur Überwachung der Körperfunktions-

0011866

- 3 -        VPA 78 P 5136 EUR

signale von erwachsenen Patienten ausgerichtet ist.
Eine allgemeine Verwendbarkeit, im besonderen für die
Überwachung von Säuglingen, ist damit jedoch nicht gewährleistet. Säuglinge im kritischen Zustand liegen
bevorzugt in Inkubatoren und anderen Reanimationseinheiten.

Aufgabe der Erfindung ist es nun, ausgehend vom genannten Stand der Technik eine Vorrichtung der eingangs genannten Art zu schaffen, mit der Herz- und
Atmungsaktionen gleichzeitig berührungslos überwacht
werden können. Die Vorrichtung soll einfach aufgebaut, universell verwendbar sein und speziell für
Säuglinge sowohl im klinischen Bereich der Überwachung
als auch außerhalb, beispielsweise im Kreißsaal oder
im häuslichen Bereich, benutzbar sein.

Die Aufgabe wird erfindungsgemäß dadurch gelöst, daß
die Lagerstatt aus einem Unterteil und einem mittels
Lagereinrichtung darauf gelagerten Oberteil mit Liegefläche für den Patienten gebildet ist, wobei die Lagerelemente zwischen Unterteil und Oberteil in wenigstens
einem Einzelelement als Kraftsignalgeber ausgebildet
sind, und daß die Lagerstatt Mittel zur Unterdrückung
von außen einwirkenden, nicht vom Patienten verursachter Kräfte beinhaltet.

Gemäß der Erfindung ist also die Lagerstatt des Patienten derart ausgebildet, daß die vom Oberteil auf
das Unterteil ausgeübten Kräfte unmittelbar erfaßt
werden. Dabei können im wesentlichen Signalspektren
aufgenommen werden, wie sie in etwa einem mechanischen
Ballistogramm entsprechen. Im Gegensatz dazu sind aber
mit der erfindungsgemäßen Vorrichtung die Signale
quasistatisch, d.h. mittels üblicher Kraftaufnehmer

- 4 -      VPA 78 P 5136  EUR

als Signalgeber, erfaßbar. Kraftsignalgeber können in geforderter Weise empfindlich und hochauflösend gewählt werden, so daß die Signalspektren mittels zugeordneter Signalverarbeitungsglieder in die interessierenden Frequenzbänder ausgefiltert werden können. Damit stehen Signale für Atmung und Herzaktion zur Verfügung.

Gemäß der Erfindung beinhaltet die Lagerstatt Mittel zur Unterdrückung der von außen einwirkenden Störungen, insbesondere zur Dämpfung der mechanischen Krafteinwirkungen aus der Umgebung. Vorzugsweise sind diese Mittel als Dämpfungselemente im Unterteil ausgebildet. Solche Elemente sind insbesondere dann notwendig, wenn die Lagerstatt in Inkubatoren eingesetzt werden soll, die bekanntermaßen starke Eigenschwingungen aufweisen. Als Dämpfungselemente haben sich beispielsweise pneumatische Bauteile, wie elastische Schläuche mit Überdruckfüllung, oder auch mechanische Bauteile, wie Stoßdämpfer oder elastomere Schaumstoffe, bewährt.

Das Oberteil mit Liegefläche der erfindungsgemäßen Vorrichtung ist im wesentlichen als schalenförmiges Formteil mit hochgezogenem Rand, vorzugsweise als Wanne für einen Säugling, ausgebildet. Sie besteht vorzugsweise aus Kunststoffmaterial mit glatter Oberfläche, z.B. schlagfestem Polystyrol oder glasfaserverstärktem Polyester. Solche abnehmbaren Kunststoffteile können bei Gebrauch schnell separat von Körperausscheidungen gesäubert sowie anschließend desinfiziert und sterilisiert werden. Besonders vorteilhaft ist dabei auch die Röntgendurchlässigkeit des Kunststoffes. Eine solche abnehmbare Wanne kann also gleichzeitig auch als Transportliege für den Patienten benutzt werden.

Die Lagerung des Oberteils auf dem Unterteil mittels der Lagerelemente ist vorzugsweise als Dreipunktlagerung ausgebildet. Dabei bildet die Verbindung der beiden ersten Lagerpunkte die Grundseite eines gleichschenkeligen Dreiecks, die parallel zu der einen kurzen Seitenkante der Liegefläche verläuft, während das dritte Lager in der Mitte nahe gegenüberliegenden anderen kurzen Seitenkanten liegt. Im dritten Lager ist als Kraftsignalgeber ein Element aus piezoelektrischem Material angeordnet, auf das über einen Stempel die Kräfte des Oberteils der Lagerstatt einwirken. Die beiden ersten Lager an der Dreiecksbasis können aus elastischem Material, z.B. Silikongummi, gebildet werden. Es ist jedoch auch eine feste Lagerung in diesen beiden Punkten möglich. Bei einer solchen festen Lagerung wird an den beiden ersten Lagerpunkten das Oberteil entweder mechanisch oder auch magnetisch am Unterteil fixiert. Insbesondere bei magnetischer Fixierung ist bei Bedarf ein schnelles Abnehmen des Oberteils vom Unterteil gewährleistet.

Weitere Einzelheiten und Vorteile der erfindungsgemäßen Vorrichtung ergeben sich aus der nachfolgenden Figurenbeschreibung von Ausführungsbeispielen anhand der Zeichnung in Verbindung mit weiteren Unteransprüchen. Es zeigen:

Fig. 1 eine Seitenansicht eines ersten Ausführungsbeispiels,

Fig. 2 die Draufsicht des Ausführungsbeispiels nach Fig. 1,

Fig. 3 die Draufsicht auf ein Unterteil eines zweiten Ausführungsbeispiels,

Fig. 4 einen Schnitt an zwei Stellen IVa und
IVb der Fig. 3 in vergrößerter Darstellung
und

Fig. 5 eine Schnittdarstellung eines weiteren Ausführungsbeispiels mit zusätzlichem zweiten
Kraftsignalgeber.

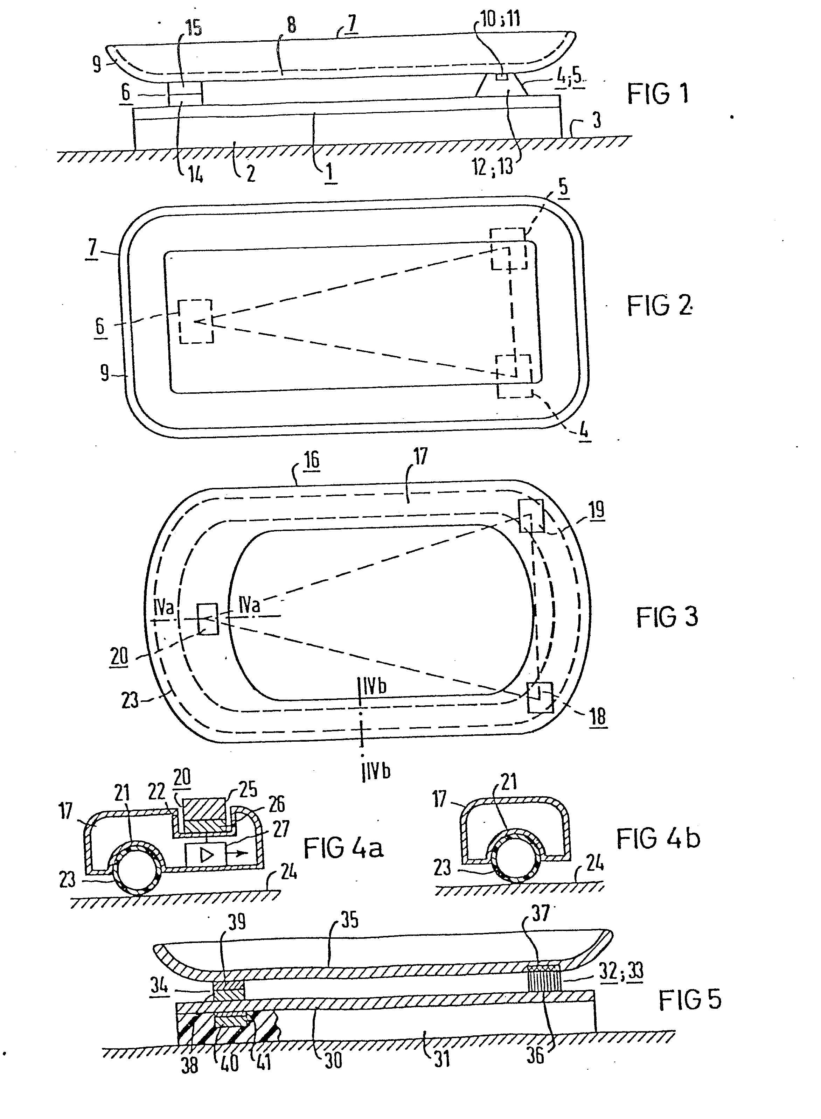

In der Fig. 1 ist mit 1 ein Unterteil bezeichnet, das unter Zwischenlegung einer Schaumstoffmatte 2 auf einer Unterlage 3, beispielsweise in einem Inkubator, gelagert ist. Am Unterteil 1 sind an einem Ende zwei Lagereinrichtungen 4 und 5 in hintereinanderliegenden Ebenen und am anderen Ende eine Lagereinrichtung 6 in der Mitte angeordnet. Mittels dieser Lagereinrichtungen, die im einzelnen noch beschrieben werden, ist ein Oberteil 7 mit Liegefläche 8 für einen Säugling auf dem Unterteil 1 gelagert.

Aus der Draufsicht nach Fig. 2 erkennt man die im wesentlichen rechteckförmige Grundfläche des Oberteils 7 mit abgerundeten Ecken. Das Oberteil 7 ist als Schale aus Kunststoffmaterial mit glatter Oberfläche als Liegefläche 8, beispielsweise aus schlagfestem Polystyrol oder glasfaserverstärktem Polyester, ausgebildet. Solche Materialien können leicht in die geeignete Form gebracht werden. Mit einem hochgezogenen Rand 9 bildet das Oberteil in etwa eine Wanne. Vor und bei Gebrauch der Wanne 7 als Liegefläche 8 für einen Säugling kann jederzeit eine Säuberung von Körperausscheidungen mit notwendiger Desinfizierung und Sterilisierung durchgeführt werden. In einer solchen Wanne 7 ist ein Säugling leicht transportierbar. Notwendige Untersuchungen, wie Röntgenaufnahmen, können daher ohne lästige Umbettung durchgeführt werden.

Aus der Fig. 2 ist die Anordnung der Lagerpunkte als gleichschenkeliges Dreieck im einzelnen ersichtlich. Am Oberteil 7 sind an den beiden Lagerpunkten 4 und 5 integrierte starre Blöcke 10 und 11 angebracht, die in Ausnehmungen von Gegenelementen 12 und 13 aus elastischem Kunststoff, z.B. Silikongummi, auf dem Unterteil 1 einrasten.

Das Lager 6 beinhaltet in seinem unteren Teil einen Kraftaufnehmer 14 als Signalgeber. Zur Erzielung eines möglichst guten Kraftschlusses in Richtung auf den Kraftaufnehmer 14 ist ein Stempel 15 über dem Kraftaufnehmer 14 angeordnet. Das Oberteil 7 liegt mit seiner Unterseite auf der Oberfläche des Stempels 15 auf.

Als Kraftaufnehmer 14 werden in beispielhafter Ausführungsform der Erfindung piezoelektrische Keramiken verwendet. Als geeignet hat sich dabei Bleizirkonattitanat (Pb[ZrTi]$O_3$) erwiesen. Die piezoelektrische Ladungskonstante in Vorzugsrichtung beträgt für ein solches Material $d_{33} = 146 \cdot 10^{-12}$ C/N. Zur Aufnahme der wirksamen Kräfte genügt eine piezoelektrische Keramik von 1 mm Stärke in Vorzugsrichtung. Die Grundfläche des Keramikplättchens 14 beträgt im Ausführungsbeispiel 5 x 5 mm.

In der Fig. 3 ist mit 16 ein etwa ellipsenförmiges Unterteil gekennzeichnet, das aus einem starren Rahmen 17 und einem darin integrierten Dämpfungskörper besteht. Wie in Fig. 2 sind mit 18 bis 20 wiederum die ein gleichschenkeliges Dreieck bildenden Lagerpunkte bezeichnet. Angedeutet sind zwei Schnittstellen IVa und IVb durch den starren Rahmen 17.

In der Schnittdarstellung nach Fig. 4a bedeutet 17 den starren Rahmen nach Fig. 3 mit einer halbkreisförmigen Ausnehmung 21 an der Unterseite und einer rechteckigen Ausnehmung 22 an der Oberseite. In die untere Ausnehmung 21 ist ein luftgefüllter Schlauch 23, in die obere Ausnehmung 22 eine Lagereinrichtung 20, bestehend aus Stempel 25 und Kraftaufnehmer 26, eingelassen.

Eine im wesentlichen gleiche Ansicht bezüglich der Lagerung des Schlauches 23 ergibt sich aus der Fig. 4b.

Das Unterteil 16 liegt also nicht direkt, sondern über den ringförmigen luftgefüllten Schlauch 23 auf dem Untergrund 24 auf. Dadurch ist eine Abdämpfung von äußeren mechanischen Wirkungen erreicht.

In der Fig. 4a wird das vom Kraftaufnehmer 26 erzeugte elektrische Signal auf einen Ladungsverstärker 27 gegeben und anschließend elektronisch im Sinne der Aufteilung des Signalspektrums in einzelne Frequenzbänder weiterverarbeitet. Die elektronischen Baugruppen zur Weiterverarbeitung (nicht eingezeichnet) können ebenfalls im Unterteil 16 angeordnet sein.

Im Ausführungsbeispiel nach Fig. 1 und 2 erfolgt die Abdämpfung der Umgebungseinflüsse durch die Schaumstoffmatte 2. Bei Verwendung von Polyäther-Schaumstoff mit 30 mm Stärke konnten bei experimentellen Untersuchungen Störungen im Frequenzbereich von 35 bis 200 Hz um etwa 20 dB vermindert werden. Im Ausführungsbeispiel nach Fig. 3 und 4 erfolgt die Abdämpfung dagegen pneumatisch. Dafür wird der Schlauch 23 aus elastomerem Material mit Überdruck von etwa 0,2 bar gefüllt. Mit einer solchen Anordnung konnten bei experimentellen

- 9 -        VPA 78 P 5136 EUR

Untersuchungen Störungen im Bereich von 35 bis 200 Hz bis zu 50 dB vermindert werden.

Das Ausführungsbeispiel nach Fig. 5 entspricht vom Aufbau her im wesentlichen dem nach Fig. 1: Ein Unterteil 30 ist auf einer Schaumstoffmatte 31 gelagert und hat Lagereinrichtungen 32 bis 34, auf denen ein Oberteil 35 gelagert ist. Die Lagereinrichtungen 32 und 33 sind in diesem Ausführungsbeispiel als starre Magnetverbindungen ausgebildet. Dazu wird als unterer Lagerblock ein Permanentmagnet 36 auf dem Unterteil 30 befestigt, während im Oberteil 35 ein Gegenelement 37 eingelassen ist. Solche Magnetverbindungen sind stabil und sicher und können von der Bedienungsperson mit zumutbarem Kraftaufwand gelöst werden. Das Lagerelement 34 beinhaltet dagegen wiederum ein Piezoplättchen 38 als Kraftaufnehmer mit zugehörigem Stempel 39. Zwischen Unterteil 30 und Dämpfungsplatte 31 ist entsprechend Fig. 5 ein zusätzlicher Kraftaufnehmer 40 mit Auflagestempel 41 in die Dämpfungsplatte 31 eingelassen, dessen Eigenschaften denen des Kraftaufnehmers im Lager 34 entsprechen. Mit diesem zweiten Kraftaufnehmer 40 wird ein durch mechanische Einwirkungen aus der Umgebung verursachtes Signal erzeugt. Dieses Signal kann als Kompensationssignal für die Verarbeitungsschaltung verwendet werden.

Der zusätzliche Kraftaufnehmer kann auch in einem der beiden Lagerelemente 32 oder 33 angeordnet sein. In einem solchen Fall wird ein gegensinniges Kraftsignal erzeugt. Bei geeigneter Meßwertverarbeitung lassen sich damit ebenso unerwünschte Artefakte eliminieren.

Besonders vorteilhaft ist bei den erfindungsgemäßen Vorrichtungen, daß die Signale praktisch lage- und ge-

0011866

wichtsunabhängig erzeugt werden. Aus diesem Grund können die überwachten Patienten im Gegensatz zu den bisher bekannten Vorrichtungen gewichtsmäßig leichte Säuglinge, z.B. auch Frühgeborene, sein. Natürlich kann die Erfindung auch für die Überwachung aller anderen Patientengruppen angewandt werden, wobei sich die gleichen günstigen Ergebnisse ergeben.

Es ist weiterhin vorteilhaft, daß der Patient während der Überwachung mit keinem Signalverarbeitungsgerät in Kontakt steht und dadurch keinen Gefährdungen durch elektrische Ströme ausgesetzt ist.

0011866

- 1 -        VPA 78 P 5136 EUR

Patentansprüche

1. Vorrichtung zur Erfassung von Körperfunktionen von Patienten, insbesondere von Säuglingen, in Verbindung mit mechanischen Einwirkungen auf eine Lagerstatt für den Patienten, die mittels Kraftsignalgeber und zugeordneten Verarbeitungsgliedern erfaßbar sind, d a d u r c h   g e k e n n z e i c h n e t ,   daß die Lagerstatt aus einem Unterteil (1, 16, 30) und einem mittels Lagereinrichtung darauf gelagerten Oberteil (7, 35) mit Liegefläche (8) für den Patienten gebildet ist, wobei die Lagerelemente (4 bis 6, 18 bis 20, 32 bis 34) zwischen Unterteil (1, 16, 30) und Oberteil (7, 35) in wenigstens einem Einzelelement als Kraftsignalgeber (14, 15, 25, 26, 38 bis 41) ausgebildet sind, und daß die Lagerstatt Mittel zur Unterdrückung von außen einwirkender, nicht vom Patienten verursachter Kräfte beinhaltet.

2. Vorrichtung nach Anspruch 1, d a d u r c h   g e k e n n z e i c h n e t ,   daß Unterteil (1, 16,30) und Oberteil (7, 35) mit Liegefläche (8) voneinander trennbar sind.

3. Vorrichtung nach Anspruch 1 und 2, d a d u r c h   g e k e n n z e i c h n e t ,   daß das Oberteil (7,35) mit Liegefläche (8) ein schalenförmiges Formteil mit hochgezogenem Rand (9) ist, das in seinen Abmessungen auf die Körpermaße des Patienten abgestimmt ist.

4. Vorrichtung nach Anspruch 2 und 3, d a d u r c h   g e k e n n z e i c h n e t ,   daß das Oberteil (7,35) eine Wanne zur Aufnahme eines Säuglings ist.

5. Vorrichtung nach Anspruch 2 bis 4, d a d u r c h  g e k e n n z e i c h n e t , daß Oberteil (7, 35) mit Liegefläche (8) aus einem Kunststoffmaterial mit glatter Oberfläche gebildet ist, beispielsweise schlagfestes Polystyrol oder glasfaserverstärktes Polyester.

6. Vorrichtung nach Anspruch 1 bis 5, d a d u r c h  g e k e n n z e i c h n e t ,  daß das Oberteil (7,35) mit Liegefläche (8) auf dem Unterteil (1, 16, 30) mit Lagerelementen in drei Punkten in Form eines gleichschenkeligen Dreiecks gelagert ist.

7. Vorrichtung nach Anspruch 1 und 2, d a d u r c h  g e k e n n z e i c h n e t ,  daß speziell das Unterteil (1., 16, 30) Elemente (2, 23, 31) zur Dämpfung mechanischer Einwirkungen aus der Umgebung aufweist.

8. Vorrichtung nach Anspruch 1 und 7, d a d u r c h  g e k e n n z e i c h n e t , daß das Unterteil (1,16, 30) als Rahmen (17) ausgebildet ist, der Ausnehmungen(21,22) für Dämpfungselemente (23) einerseits und Lagerelemente (18 bis 20) andererseits hat.

9. Vorrichtung nach Anspruch 7, d a d u r c h  g e k e n n z e i c h n e t , daß das Dämpfungselement ein pneumatisches Bauteil (23) ist, beispielsweise ein elastischer Schlauch mit Überdruckfüllung.

10. Vorrichtung nach Anspruch 7, d a d u r c h  g e k e n n z e i c h n e t ,  daß das Dämpfungselement ein Schaumstoff (2, 31) mit elastomeren Eigenschaften ist, beispielsweise eine Platte aus weichgeschäumtem Polyäther.

0011866

11. Vorrichtung nach Anspruch 7, d a d u r c h g e k e n n z e i c h n e t , daß das Dämpfungsmittel ein mechanischer Stoßdämpfer, beispielsweise ein Federelement od.dgl., ist.

12. Vorrichtung nach Anspruch 6, d a d u r c h g e k e n n z e i c h n e t , daß die beiden ersten Lagerelemente (4, 5) Blöcke (12, 13) aus elastischem Material, z.B. Silikongummi, sind.

13. Vorrichtung nach Anspruch 6, d a d u r c h g e k e n n z e i c h n e t , daß das Oberteil (7,35) an den beiden ersten Lagerpunkten (32, 33) fest auf dem Unterteil (1, 16, 30) aufliegt.

14. Vorrichtung nach Anspruch 2 und 13, d a - d u r c h g e k e n n z e i c h n e t , daß Oberteil (7, 35) und Unterteil (1, 16, 30) im Bereich der ersten Lager (18, 19, 32, 33) mechanisch und/oder magnetisch fixiert sind.

15. Vorrichtung nach Anspruch 13 und 14, d a - d u r c h g e k e n n z e i c h n e t , daß das Oberteil (7, 35) im Bereich der Lagerelemente (18, 19, 32, 33) Gegenelemente (37) für Magnetverbindungen aufweist.

16. Vorrichtung nach Anspruch 1, d a d u r c h g e k e n n z e i c h n e t, daß der Kraftsignalgeber (15, 26, 38, 40) ein Keramikelement aus piezoelektrischem Material, z.B. Bleizirkonattitanat, ist.

17. Vorrichtung nach Anspruch 6 und 16, d a - d u r c h g e k e n n z e i c h n e t , daß das piezoelektrische Keramikelement im dritten Lager (6,20, 34) angeordnet ist, wobei über einen Stempel (14,25,38)

- 4 -        VPA 78 P 5136  EUR

od.dgl. die auf das Oberteil (7, 16, 35) mit Liegefläche (8) wirkenden Kräfte quasistatisch auf das
Keramikelement übertragen werden.

18. Vorrichtung nach Anspruch 6,  d a d u r c h
g e k e n n z e i c h n e t ,  daß in einem der
ersten Lagerelemente (4, 5, 19, 20) ein weiterer Kraftsignalgeber angeordnet ist, mit dem ein zum ersten
Kraftsignalgeber (15, 26, 38) gegensinniges Signal
erzeugt wird.

19. Vorrichtung nach Anspruch 1 und 8,  d a d u r c h
g e k e n n z e i c h n e t ,  daß ein weiterer
Kraftsignalgeber (40) zwischen Unterteil (30) der Lagerstatt und Dämpfungselement (31) angeordnet ist.

20. Vorrichtung nach Anspruch 1,  d a d u r c h
g e k e n n z e i c h n e t ,  daß dem Kraftsignalgeber zugeordnete Verarbeitungsglieder, z.B. ein
Ladungsverstärker (27) sowie weitere elektronische
Baugruppen zur Weiterverarbeitung der elektrischen
Signale im Unterteil (1, 16, 30), angeordnet sind.

FIG 1

FIG 2

FIG 3

FIG 4a

FIG 4b

FIG 5

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | DE - A1 - 2 515 231 (MOOG AUTOM. INC.)<br><br>+ Seite 7, 2. Absatz bis Seite 9, 1. Absatz; Seite 10, 3. Absatz bis Seite 12, 3. Absatz; Seite 18, 2. Absatz bis Seite 21, 2. Absatz; Seite 25, 2.Absatz bis Seite 32, 2. Absatz; Fig. 1-9,12-16 +<br><br>-- | 1,2,6, 7,10, 13,14, 16,17, 20 |
| X | CH - A - 324 337 (Dr.U.A. CORTI)<br><br>+ Gesamt +<br><br>-- | 1,18, 20 |
| X | US - A - 3 906 931 (Y.V. TEREKHOV)<br><br>+ Spalte 3, Zeile 30 bis Spalte 4, Zeile 23; Spalte 5, Zeilen 15 bis 48; Fig. 1,2 +<br><br>-- | 1,2, 18,20 |
| | FR - A - 1 442 508 (CENTRE NAT.)<br><br>+ Gesamt +<br><br>-- | 1 |
| A | DE - A - 2 345 551 (SPEARHEAD INC.)<br><br>+ Seite 7, 6. Absatz - Seite 8, 2. Absatz; Patentanspruch 1; Fig. 1 +<br><br>-- | 1 |
| A | DD - A - 106 133 (Dr.G.MLETZKO)<br><br>+ Gesamt +<br><br>-- | 1 |
| A | DE - A - 2 106 275 (SIEMENS)<br><br>+ Gesamt +<br><br>---- | |

### KLASSIFIKATION DER ANMELDUNG (Int.Cl.X) 3

A 61 B 5/10
A 61 B 5/00

### RECHERCHIERTE SACHGEBIETE (Int. Cl.X 3

A 61 B 5/00
A 61 G 7/00

### KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patent- familie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| WIEN | 29-02-1980 | LUDWIG |

EPA form 1503.1   06.78